# EUROPEAN PATENT SPECIFICATION

(11) **EP 1 611 892 B1**
(45) Date of publication and mention of the grant of the patent: **09.09.2009**
(21) Application number: 05076969.4
(22) Date of filing: 18.01.2001
(51) Int. Cl.: A61K 31/585, A61P 33/00

(54) **Pharmaceutical compositions comprising drospirenone**
Pharmazeutische Zubereitung enthaltend Drospirenon
Compositions pharmaceutiques comprenant du drospirenone

(30) Priority: 18.01.2000 EP 00200183; 18.01.2000 US 484026
(43) Date of publication of application: 04.01.2006
(62) Divisional of application: 01900579.2
(73) Proprietor: Bayer Schering Pharma Aktiengesellschaft, 13353 Berlin (DE)
(72) Inventor: Heil, Wolfgang, 21435 Stelle (DE); Hilmann, Juergen, 13351 Berlin (DE); Lipp, Ralph, Zionsville, IN 46077 (US); Schuermann, Rolf, D-14513 TELTOW (DE)
(74) Representative: Plougmann & Vingtoft A/S

(56) References cited:
- WO-A-01/15701
- WO-A-98/27929
- MUHN P ET AL: "DROSPIRENONE: A NOVEL PROGESTOGEN WITH ANTIMINERALOCORTICOID AND ANTIANDROGENIC ACTIVITY PHARMACOLOGICAL CHARACTERIZATION IN ANIMAL MODELS" CONTRACEPTION, GERON-X, INC., LOS ALTOS, CA, US, vol. 51, no. 2, February 1995 (1995-02), pages 99-110, XP000908817 ISSN: 0010-7824

## Description

### FIELD OF INVENTION

The present invention relates to a pharmaceutical composition comprising drospirenone.

### GENERAL BACKGROUND OF THE INVENTION

Drospirenone is known from DE 26 52 761 in which its use as a diuretic is disclosed.

The gestagen-like activity of drospirenone and its consequent utility as a contraceptive agent at dosage levels of 0.5-50 mg is disclosed in DE 30 22 337.

The use and role of progestogens in opposed forms of hormone replacement therapy has been studied by the scientific community (Lobo R.A., 1992; Sobel N.B., 1994) as have been regimens comprising estrogens and progestogens (Corson S.L., 1993; Jones K.P., 1992).

The use of a preparation for substitution therapy and for oral contraception comprising at least one progestagen and at least one estrogen wherein the estrogen dose varies with a periodicity such that blood toss is substantially avoided is disclosed in PCT/EP94/02997.

### SUMMARY OF THE INVENTION

The invention relates in a first aspect to a pharmaceutical composition comprising drospirenone together with a pharmaceutically acceptable carrier of excipient, wherein said composition does not contain an estrogen and wherein said drospirenone is in micronised form.

In a second aspect, the present invention relates to a pharmaceutical composition comprising drospirenone together with a pharmaceutically acceptable carrier of excipient, wherein said composition does not contain an estrogen and wherein said drospirenone is in a form having a surface area of more than 10,000 cm²/g.

In a further aspect, the present invention relates to a pharmaceutical composition comprising drospirenone together with a pharmaceutically acceptable carrier of excipient, wherein said composition does not contain an estrogen and wherein said drospirenone is in a form having rapid dissolution such that at least 70% of said drospirenone is dissolved within 30 minutes when the composition is subjected to dissolution testing in 900 ml of water at 37°C using USP XXIII Paddle Method II operated at a stirring rate of 50 rpm.

In an even further aspect, the present invention relates to a pharmaceutical composition comprising inert carrier particles containing drospirenone on their surface, wherein said composition does not contain an estrogen, and wherein at least 70% of said drospirenone is dissolved within 30 minutes when the composition is subjected to dissolution testing in 900 ml of water at 37°C using USP XXIII Paddle Method II operated at a stirring rate of 50 rpm.

In a still further aspect, the present invention relates to a pharmaceutical preparation consisting of a number of separately packaged and individually removable dosage units according to the invention placed in a packaging unit and intended for oral administration for a period of at least 21 days.

In yet another aspect, the present invention relates to the use of a composition according to the invention for the manufacture of a medicament for protecting the endometrium from hyperplasia or cancer.

Moreover, the present invention relates to the use of a composition according to the invention for the manufacture of a medicament for the treatment of endometriosis.

### DETAILED DISCLOSURE OF THE INVENTION

The use of natural progesterone in therapy is limited by the low bioavailability of natural progesterone, even in micronized form. Significantly, it has been found that therapy comprising the use of drospirenone as a progestagen, is remarkably effective. Drospirenone (DRSP), a 17-α-spirotactone derivative, is a synthetic progestagen that has a surprisingly similar physiological profile to progesterone yet notably better bioavailability. It is the first synthetic progestagen to have a progesterone-like pharmacological profile in that it is antiestrogenic, antiandrogenic, and has antimineralcorticoid activity.

A part form the active substance itself, it is envisaged that an ester or prodrug of drospirenone may be emptoyed in the present composition, e.g. an oxyiminopregnane carbolactone as disclosed in WO 98/24801.

An interesting embodiment of the invention comprises a composition wherein the drospirenone (DRSP) is in micronized form.

Drospirenone, which may be prepared substantially as described in, e.g., US 4,129,564 or WO 98/06738, is a sparingly soluble substance in water and aqueous buffers at various pH values. Furthermore, drospirenone is rearranged to an inactive isomer under acid conditions and hydrolysed under alkaline conditions. To ensure good bioavailability of the compound, it is therefore advantageously provided in a form that promotes rapid dissolution thereof.

It has been found that when drospirenone is provided in micronized form in a pharmaceutical composition, rapid dissolution of the active compound from the composition occurs *in vitro.* A micronized substance is such that a test batch (ca. 200 mg) of the particles, herein drospirenone particles, has a surface area of more than 10,000 cm²/g, and has the following particle size distribution for drospirenone as determined under the microscope: not more than 2 particles in a given batch (ca. 200 mg) with a diameter of more than 30 µm, and preferably ≤ 20 particles with a diameter of ≥10 µm and ≤ 30 µm. The term "rapid dissolution" is defined as the dissolution of at least 70% over about 30 minutes, in particular at least 80% over about 20 minutes, of drospirenone from a tablet preparation containing 3 mg of drospirenone in 900 ml of water at 37°C determined by the USP XXIII Paddle Method using a USP dissolution test apparatus 2 at 50 rpm.

As an alternative to providing the drospirenone in micronized form, it is possible to dissolve it in a suitable solvent, e.g. methanol or ethyl acetate, and spray it onto the surface of inert carrier particles followed by incorporation of the particles containing drospirenone on their surface in the composition.

Without wishing to be limited to any particular theory, it appears that the *in vitro* dissolution rate of drospirenone is connected to the dissolution rate *in vivo* resulting in rapid absorption of drospirenone *in vivo* on oral administration of the compound. This is an advantage because isomerization of the compound in the gastric environment and/or hydrolysis in the intestine is substantially reduced, leading to a high bioavailability of the compound.

To obtain a more rapid rate of dissolution, it is preferred to include carriers or excipients, which act to promote dissolution of the active substance. Examples of such carriers and excipients include substances that are readily soluble in water such as cellulose derivatives, carboxymethyl cellulose, hydroxypropyl cellulose, hydroxypropyl methyl cellulose, gelled starch, gelatin or polyvinylpyrrolidone. In particular, it is anticipated that polyvinylpyrrolidone might be particularly helpful to promote dissolution.

The dose of the drospirenone in each composition is preferably such as to protect the endometrium from the adverse effects of estrogen. DRSP, in sufficient doses, may be used as an opponent to estrogen to protect the endometrium form hyperplasia or cancer.

In some cases, however, the dose of DRSP may be sufficient so as to stabilise the menstrual cycle and bleeding pattern. Adipose tissue production of estrogen may be such that no estrogen is required to stabilise the menstrual cycle and bleeding pattern. Moreover, in embodiments, where the woman suffers from other disorders not compatible with the use of an exogenous source of estrogen (such as for the absolute contraindications of severe liver diseases and pregnancy or for the relative contraindications endometrial carcinoma and endometriosis, mammary carcinoma, venous thromboembolism, hypertension, diabetes mellitus, otosclerosis and melanoma) no estrogen is in the composition. In such embodiments, the dose of DRSP may be such that the disorder, disease, or symptom is alleviated.

In preferred embodiments, the dose of DRSP corresponds to 15 to 70 mg per cycle, such as 20 to 60 mg per cycle, particularly 40 to 60 mg per cycle. The length of the cycle may vary from 21 to 31 days. Viewed otherwise, a composition may comprise an amount of DRSP corresponding to a daily dose ranging from 0.25 to 10, such as about 0.25 to 8, 0.25 to 6, 0.25 to 5, 0.5 to 4.5, 1 to 4, and 1.5 to 3.5 mg.

In other embodiments, the dose of DRSP is sufficient to treat hot flushes, sweating attacks, palpitations, sleep disorders, mood changes, nervousness, anxiety, poor memory, toss of confidence, loss of libido, poor concentration, diminished energy, diminished drive, irritability, urogenital atrophy, atrophy of the breasts, cardiovascular disease, changes in hair distribution, thickness of hair, changes in skin condition or for the prevention or management of osteoporosis.

The dose of DRSP may depend on the treatment, be it for endometrial protection such as bleeding pattern, for osteoporosis prevention or management, for the treatment of menopausal symptoms, such as the for the reduction in the number, frequency, and severity of hot flushes, night sweats, mood swings palpitations, insomnia and other sleep disorders, mood changes, nervousness, anxiety, poor memory, loss of confidence, loss of libido, poor concentration, diminished energy, diminished drive and irritability.

In embodiments where the woman is peri-menopausal the dose of DRSP may depend on the day of the cycle, that is, whether she is in the preovulatory or postovulatory phase of the cycle, and how advanced within each phase. In embodiments where the woman is post-menopausal or even pre-menopausal, the dose of DRSP may depend on the time since the last menstruation.

In certain embodiments of the invention, the medicament is administered in the form a of a number of separately packaged and individually removable dosage units placed in a packaging unit and Intended for oral administration for a period of at least 21, such as at least 28 days, such as at least 30 or 31 days. In such embodiments, the dose of DRSP may be the same within each dosage unit or may vary. In embodiments where the amount of DRSP in the dosage unit varies according to the phase or day within the period of at least 21 days, such as at least 28 days that said dosage unit is to be administered, such compositions, methods of treatments and preparations are termed multi-phased.

The composition of the dosage unit may be formulated in any way conventional in the pharmaceutical art. In particular, as indicated above, the composition may be formulated by a method comprising providing drospirenone in micronized form in said unit dosage form, or sprayed from a solution onto particles of an inert carrier in admixture with one ormore pharmaceutically acceptable excipients that promote dissolution of the drospirenone so as to promote rapid dissolution of drospirenone on oral administration. Examples of suitable excipients include fillers, e.g. sugars such as lactose, glucose or sucrose, sugar alcohols such as mannitol, starch such as corn or potato starch or modified starch, lubricants such as talc or magnesium stearate and binders such as polyvinylpyrrolidone, cellulose derivatives, carboxymethyl cellulose, hydroxypropyl cellulose, hydroxypropylmethyl cellulose, methyl cellulose, or gelatin for making oral dosage forms such as tablets, pills or capsules. Tablet may conveniently be coated with a suitable film-forming agent, e.g. hydroxypropylmethylcellulose. The present composition may also be formulated in liquid form, e.g. as solutions, suspensions or emulsions, together with conventional diluents or excipients in a manner known per se in the pharmaceutical art.

A packaging unit comprising the daily dosage units described above may be prepared in a manner analogous to that of making oral contraceptives or hormone replacement regimens. This may for instance be a conventional blister pack or any other form known for this purpose, for instance a pack comprising the appropriate number of dosage units (in this case at least 28, or for particular applications, a multiple of 28) in a sealed blister pack with a cardboard, paperboard, foil or plastic backing and enclosed in a suitable cover. Each blister container may conveniently be numbered or otherwise marked.

it is also envisaged that the present composition may be in the form of a parenteral formulation such as a subcutaneous implant or transdermal formulation. For making implants, the active agent may suitably be formulated together with one or more polymers that are gradually eroded or degraded when in use, e.g. silicone polymers, ethylene vinylacetate, polyethylene or polypropylene.

Where transdermal formulations are concerned, they may be prepared in the form of matrices or membranes or as fluid or viscous formulations in oil or hydrogels. For transdermal patches, an adhesive which is compatible with the skin should be included, such as polyacrylate, a silicone adhesive or polylisobutylene, as well as a foil made of, e.g. polyethylene, polypropylene, ethylene vinylacetate, polyvinylchloride, polyvinylidene chloride or polyester, and a removable protective foil made from, e.g., polyester or paper coated with silicone or a fluoropolymer. For the preparation of transdermal solutions or gels, water or organic solvents or mixtures thereof may be used. Transdermal gets may furthermore contain one or more suitable gelling agents or thickeners such as silicone, tragacanth, starch or starch derivatives, cellulose or cellulose derivatives or polyacrylic acids or derivatives thereof. Transdermal formulations may also suitably contain one or more substances that enhance absorption though the skin, such as bile salts or derivatives thereof and/or phospholipids. Suitable transdermal formulations may, for instance, be made in a manner analogous to that described in WO 94/04157 for 3-ketodesogestrel. Alternatively, transdermal formulations may be prepared according to a method disclosed in, e.g., BW Barry, "Dermatological Formulations, Percutaneous Absorption", Marcel Dekker Inc., New York - Basel, 1983, or YW Chien, "Transdermal Controlled Systemic Medications". Marcel Dekker Inc., New York - Basel. 1987.

## Claims

1. A pharmaceutical composition comprising drospirenone together with a pharmaceutically acceptable carrier of excipient, wherein said composition does not contain an estrogen and wherein said drospirenone is in micronised form.

2. A pharmaceutical composition comprising drospirenone together with a pharmaceutical acceptable carrier of excipient, wherein said composition does not contain an estrogen and wherein said drospirenone is in a form having a surface area of more than 10,000 cm²/g.

3. A pharmaceutical composition comprising drospirenone together with a pharmaceutically acceptable carrier of excipient, wherein said composition does not contain an estrogen and wherein said drospirenone is in a form having rapid dissolution such that at least 70% of said drospirenone is dissolved within 30 minutes when the composition is subjected to dissolution testing in 900 ml of water at 37 °C using USP XXIII Paddle Method II operated at a stirring rate of 50 rpm.

4. A pharmaceutical composition comprising inert carrier particles containing drospirenone on their surface, wherein said composition does not contain an estrogen, and wherein at least 70% of said drospirenone is dissolved within 30 minutes when the composition is subjected to dissolution testing In 900 ml of water at 37 °C using USP XXIII Paddle Method II operated at a stirring rate of 50 rpm.

5. The composition according to claim 4, wherein drospirenone is sprayed from a solution onto said inert carrier particles.

6. The composition according to any of the preceding claims, wherein at least 80% of said drospirenone is dissolved from said composition within 20 minutes.

7. The composition according to any of the preceding claims, wherein said composition contains drospirenone in an amount corresponding to a daily dose ranging from 0.25 to 10 mg.

8. The composition according to claim 7, wherein said composition contains drospirenone in an amount corresponding to a daily dose ranging from 0.5 to 4.5 mg.

9. The composition according to claim 8, wherein said composition contains drospirenone in an amount corresponding to a daily dose ranging from 1.5 to 3.5 mg.

10. The composition according to any of the preceding claims, wherein said composition contains a filler.

11. The composition according to claim 10, wherein said filter is a sugar, a sugar alcohol or a starch.

12. The composition according to claim 11, wherein said sugar is selected from the group consisting of lactose, glucose and sucrose.

13. The composition according to any of the preceding claim, wherein said composition contains a binder.

14. The composition according to claim 13, wherein said binder is selected from the group consisting of polyvinylpyrrolidone, carboxymethyl cellulose, hydroxypropyl cellulose, hydroxypropylmethyl cellulose, methyl cellulose and gelatin.

15. The composition according to claim 14, wherein said binder is polyvinylpyrrolidone.

16. The composition according to any of the preceding claims, wherein said composition contains a lubricant.

17. The composition according to claim 16, wherein said lubricant is talc or magnesium stearate.

18. The composition according to any of the preceding claims, wherein said composition is in the form of an oral dosage form.

19. The composition according to claim 18, wherein said oral dosage form is in the form of a tablet, a capsule or a pill.

20. The composition according to claim 19, wherein said oral dosage form is in the form of a tablet.

21. The composition according to claim 20, wherein said tablet is coated with a film-forming agent.

22. The composition according to claim 21, wherein said film-forming agent is hydroxypropylmethyl cellulose.

23. A pharmaceutical preparation consisting of a number of separately packaged and individually removable dosage units as defined in any of claims 18-22 placed in a packaging unit and intended for oral administration for a period of at feast 21 days.

24. Use of a composition as defined in any of claims 1-23 for the manufacture of a medicament for protecting the endometrium from hyperplasia or cancer.

25. Use of a composition as defined in any of claims 1-23 for the manufacture of a medicament for the treatment of endometriosis.

## Patentansprüche

1. Pharmazeutische Zusammensetzung, enthaltend Drospirenon zusammen mit einem pharmazeutisch verträglichen Träger oder Hilfsstoff, wobei die Zusammensetzung kein Estrogen enthält und das Drospirenon in mikronisierter Form vorliegt.

2. Pharmazeutische Zusammensetzung, enthaltend Drospirenon zusammen mit einem pharmazeutisch verträglichen Träger oder Hilfsstoff, wobei die Zusammensetzung kein Estrogen enthält und das Drospirenon in einer Form mit einer Oberfläche von mehr als 10.000 cm²/g vorliegt.

3. Pharmazeutische Zusammensetzung, enthaltend Drospirenon zusammen mit einem pharmazeutisch verträglichen Träger oder Hilfsstoff, wobei die Zusammensetzung kein Estrogen enthält und das Drospirenon in einer Form mit schneller Auflösung vorliegt, so daß bei einem Auflösungstest in 900 ml Wasser bei 37°C nach der Paddle-Methode II gemäß USP XXIII bei einer Rührgeschwindigkeit von 50 U/min innerhalb von 30 Minuten mindestens 70% des Drospirenons gelöst werden.

4. Pharmazeutische Zusammensetzung, enthaltend inerte Trägerteilchen, die auf ihrer Oberfläche Drospirenon enthalten, wobei die Zusammensetzung kein Estrogen enthält und bei einem Auflösungstest in 900 ml Wasser bei 37°C nach der Paddle-Methode II gemäß USP XXIII bei einer Rührgeschwindigkeit von 50 U/min innerhalb von 30 Minuten mindestens 70% des Drospirenons gelöst werden.

5. Zusammensetzung nach Anspruch 4, wobei Drospirenon aus einer Lösung von Drospirenon auf die inerten Trägerteilchen aufgesprüht worden ist.

6. Zusammensetzung nach einem der vorhergehenden Ansprüche, wobei innerhalb von 20 Minuten mindestens 80% des Drospirenons aus der Zusammensetzung herausgelöst werden.

7. Zusammensetzung nach einem der vorhergehenden Ansprüche, enthaltend Drospirenon in einer Menge, die einer Tagesdosis von 0,25 mg bis 10 mg entspricht.

8. Zusammensetzung nach Anspruch 7, enthaltend Drospirenon in einer Menge, die einer Tagesdosis von 0,5 mg bis 4,5 mg entspricht.

9. Zusammensetzung nach Anspruch 8, enthaltend Drospirenon in einer Menge, die einer Tagesdosis von 1,5 mg bis 3,5 mg entspricht.

10. Zusammensetzung nach einem der vorhergehenden Ansprüche, wobei die Zusammensetzung einen Füllstoff enthält.

11. Zusammensetzung nach Anspruch 10, wobei es sich bei dem Füllstoff um einen Zucker, einen Zuckeralkohol oder eine Stärke handelt.

12. Zusammensetzung nach Anspruch 11, wobei der Zucker aus der Gruppe bestehend aus Lactose, Glucose und Saccharose ausgewählt ist.

13. Zusammensetzung nach einem der vorhergehenden Ansprüche, wobei die Zusammensetzung ein Bindemittel enthält.

14. Zusammensetzung nach Anspruch 13, wobei das Bindemittel aus der Gruppe bestehend aus Polyvinylpyrrolidon, Carboxymethylcellulose, Hydroxypropylcellulose, Hydroxypropylmethylcellulose, Methylcellulose und Gelatine ausgewählt ist.

15. Zusammensetzung nach Anspruch 14, wobei es sich bei dem Bindemittel um Polyvinylpyrrolidon handelt.

16. Zusammensetzung nach einem der vorhergehenden Ansprüche, wobei die Zusammensetzung ein Gleitmittel enthält.

17. Zusammensetzung nach Anspruch 16, wobei es sich bei dem Gleitmittel um Talk oder Magnesiumstearat handelt.

18. Zusammensetzung nach einem der vorhergehenden Ansprüche, wobei die Zusammensetzung in Form einer oralen Dosisform vorliegt.

19. Zusammensetzung nach Anspruch 18, wobei die orale Dosisform in Form einer Tablette, einer Kapsel oder einer Pille vorliegt.

20. Zusammensetzung nach Anspruch 19, wobei die orale Dosisform in Form einer Tablette vorliegt.

21. Zusammensetzung nach Anspruch 20, wobei die Tablette mit einem Filmbildner überzogen ist.

22. Zusammensetzung nach Anspruch 21, wobei es sich bei dem Filmbildner um Hydroxypropylmethylcellulose handelt.

23. Pharmazeutische Zubereitung, bestehend aus einer Zahl getrennt verpackter und einzeln herausnehmbarer Dosiseinheiten gemäß einem der Ansprüche 18-22, die in einer Verpackungseinheit angeordnet und für die orale Verabreichung über einen Zeitraum von mindestens 21 Tagen vorgesehen sind.

24. Verwendung einer Zusammensetzung gemäß einem der Ansprüche 1-23 zur Herstellung eines Arzneimittels zum Schutz des Endometriums vor Hyperplasie und Krebs.

25. Verwendung einer Zusammensetzung gemäß einem der Ansprüche 1-23 zur Herstellung eines Arzneimittels zur Behandlung von Endometriose.

## Revendications

1. Composition pharmaceutique comprenant de la drospirénone conjointement avec un vecteur ou excipient pharmaceutiquement acceptable, dans laquelle ladite composition ne contient pas un estrogène et dans laquelle ladite drospirénone est sous une forme micronisée.

2. Composition pharmaceutique comprenant de la drospirénone conjointement avec un vecteur ou excipient pharmaceutiquement acceptable, dans laquelle ladite composition ne contient pas un estrogène et dans laquelle ladite drospirénone est sous une forme dont la surface excède 10 000 cm²/g.

3. Composition pharmaceutique comprenant de la drospirénone conjointement avec un vecteur ou excipient pharmaceutiquement acceptable, dans laquelle ladite composition ne contient pas un estrogène et dans laquelle ladite drospirénone est sous une forme avec une dissolution rapide de sorte qu'au moins 70 % de ladite drospirénone est dissoute en moins de 30 minutes lorsque la composition est soumise à un test de dissolution dans 900 ml d'eau à 37 °C en utilisant la méthode à palettes II selon la Pharmacopée US XXIII actionné à une vitesse d'agitation de 50 tr/min.

4. Composition pharmaceutique comprenant des particules d'un vecteur inerte contenant de la drospirénone à leur surface, dans laquelle ladite composition ne contient pas un estrogène et dans laquelle au moins 70 % de ladite drospirénone est dissoute en moins de 30 minutes lorsque la composition est soumise à un test de dissolution dans 900 ml d'eau à 37 °C en utilisant la méthode à palettes II selon la Pharmacopée US XXIII actionné à une vitesse d'agitation de 50 tr/min.

5. Composition selon la revendication 4, dans laquelle la drospirénone est vaporisée à partir d'une solution desdites particules de vecteur.

6. Composition selon l'une quelconque des revendications précédentes, dans laquelle au moins 80 % de ladite drospirénone est dissoute à partir de ladite composition en moins de 20 minutes.

7. Composition selon l'une quelconque des revendications précédentes, dans laquelle ladite composition contient de la drospirénone en une quantité qui correspond à une dose quotidienne dans la plage de 0,25 à 10 mg.

8. Composition selon la revendication 7, dans laquelle ladite composition contient de la drospirénone en une quantité qui correspond à une dose quotidienne dans la plage de 0,5 à 4,5 mg.

9. Composition selon la revendication 8, dans laquelle ladite composition contient de la drospirénone en une quantité qui correspond à une dose quotidienne dans la plage de 1,5 à 3,5 mg.

10. Composition selon l'une quelconque des revendications précédentes, dans laquelle ladite composition contient une charge.

11. Composition selon la revendication 10, dans laquelle ladite charge est un sucre, un alcool de sucre ou un amidon.

12. Composition selon la revendication 11, dans laquelle ledit sucre est choisi parmi le groupe constitué du lactose, du glucose et du sucrose.

13. Composition selon l'une quelconque des revendications précédentes, dans laquelle ladite composition contient un liant.

14. Composition selon la revendication 13, dans laquelle ledit liant est choisi parmi le groupe constitué de la polyvinylpyrrolidone, de la carboxyméthylcellulose, l'hydroxypropylcellulose, de l'hydroxypropylméthylcellulose, de la méthylcellulose et de la gélatine.

15. Composition selon la revendication 14, dans laquelle ledit liant est la polyvinylpyrrolidone.

16. Composition selon l'une quelconque des revendications précédentes, dans laquelle ladite composition contient un lubrifiant.

17. Composition selon la revendication 16, dans laquelle ledit lubrifiant est du talc ou du stéarate de magnésium.

18. Composition selon l'une quelconque des revendications précédentes, dans laquelle la forme de ladite composition est une forme pharmaceutique orale.

19. Composition selon la revendication 18, dans laquelle ladite forme pharmaceutique orale est sous la forme d'un comprimé, d'une capsule ou d'une pilule.

20. Composition selon la revendication 19, dans laquelle ladite forme pharmaceutique orale est sous la forme d'un comprimé.

21. Composition selon la revendication 20, dans laquelle ledit comprimé est recouvert d'un agent filmogène.

22. Composition selon la revendication 21, dans laquelle ledit agent filmogène est de l'hydroxypropylméthylcellulose.

23. Préparation pharmaceutique composée d'un certain nombre d'unités de dose emballées séparément et détachables individuellement selon l'une quelconque des revendications 18 à 22, placée dans une unité d'emballage et destinée à une administration orale pour une période d'au moins 21 jours.

24. Utilisation d'une composition selon l'une quelconque des revendications 1 à 23 pour la fabrication d'un médicament pour protéger l'endomètre d'une hyperplasie ou d'un cancer.

25. Utilisation d'une composition selon l'une quelconque des revendications 1 à 23 pour la fabrication d'un médicament pour le traitement d'une endométriose.
